# EUROPÄISCHE PATENTSCHRIFT

(11) **EP 1 727 888 B1**
(45) Veröffentlichungstag und Bekanntmachung des Hinweises auf die Patenterteilung: **02.02.2011**
(21) Anmeldenummer: 05715752.1
(22) Anmeldetag: 04.03.2005
(51) Int. Cl.: C12M 1/113

(54) **TRANSPORTEINRICHTUNG FÜR BIOMASSE IN EINEM FERMENTER ZUR ERZEUGUNG VON BIOGAS**
TRANSPORT DEVICE FOR BIOMASS, FOR USE IN A FERMENTER FOR GENERATING BIOGAS
DISPOSITIF DE TRANSPORT DE BIOMASS, DESTINE A ETRE UTILISE DANS UN FERMENTEUR POUR LA PRODUCTION DE BIOGAZ

(30) Priorität: 04.03.2004 DE 202004003398 U
(43) Veröffentlichungstag der Anmeldung: 06.12.2006
(73) Patentinhaber: Bekon Energy Technologies GmbH & CO. KG, 84034 Landshut (DE)
(72) Erfinder: LUTZ, Peter, 84034 Landshut (DE)
(74) Vertreter: Winter, Brandl, Fürniss, Hübner, Röss, Kaiser, Polte - Partnerschaft
(86) Internationale Anmeldenummer: PCT/EP2005/002326
(87) Internationale Veröffentlichungsnummer: WO 2005/085411

(56) Entgegenhaltungen:
- EP-A- 1 136 546
- WO-A-96/12789
- DE-A1- 3 341 691
- US-A- 5 312 754

## Beschreibung

Die Erfindung betrifft eine Transporteinrichtung für Biomasse in einem Fermenter zur Erzeugung von Biogas nach Anspruch 1 sowie einen Großfermenter zur Erzeugung von Biogas aus Biomasse nach Anspruch 9.

Bislang hat sich die Biogastechnik hauptsächlich auf die "Nassvergärung" von Gülle und/oder Bioabfällen aus dem kommunalen Bereich konzentriert, bei der die Biomasse in flüssiger, pumpbarer Form vorliegt. Anlagen und Vorrichtungen zur Erzeugung von Biogas aus Biomasse nach dem Naßgärverfahren sind z. B aus Druckschriften AT 408230 B, WO 96/12789, DE 3228391 A1, AT 361885 B und DE 19746636 A1 bekannt.

Aus der DE 3228391 A1 ist hierbei ein Faulbehälter in Form eines elastischen Schlauches bekannt, bei dem die Biomasse mittels einer künstlich erzeugten Peristaltik durch den Schlauch befördert werden. Die peristaltische Bewegung wird mittels über den Schlauch gezogene Schlingen, die zusammengezogen werden, mittels über den Schlauch gezogenen Manschetten, die mit Druckluft beaufschlagt werden oder mittels über die Länge des Schlauches verteilte Stempel, die aufeinanderfolgend in den Schlauch hineindrücken, erzeugt.

Aus der DE 19746636 A1 ist Faulbehälter in Form eines Rundsilos bekannt, wobei der eigentliche Faulbehälter im Zentrum des Rundsilos angeordnet und von einem ringförmigen Zwischenbereich umgeben ist.

Aus der AT 361885 B ist ebenfalls ein Faulbehälter in Form eines Rundsilos bekannt, der eine zylindrische Außenwand und eine zylindrische Innenwand umfaßt, wodurch ein ringförmiger Transportkanal gebildet wird. Die verflüssigte Biomasse wird zugeführt, durchfließt den Transport und wird wieder entnommen. Zusätzlich ist aus aus der AT 361885 B bekannt, zwischen der Innen- und der Außenwand eine zylindrische Mittelwand vorzusehen, wodurch ein innerer und ein äußerer Transportkanalring gebildet werden.

Nachwachsende Rohstoffe mit hohen Trockensubstanzgehalten (z.B. Maissilage) oder Festmiste können bei diesen "flüssigen" Verfahren nur in begrenztem Umfang beigemischt werden.

Die so genannte "Trockenfermentation" erlaubt es, schüttfähige Biomassen aus der Landwirtschaft, aus Bioabfällen und kommunalen Pflegeflächen zu methanisieren, ohne die Materialien in ein pumpfähiges, flüssiges Substrat zu überführen. Es können Biomassen mit bis zu 50% Trockensubstanzanteil vergoren werden. Dieses Trockenfermentations-Verfahren ist beispielsweise in der EP 0 934 998 beschrieben.

Bei der "trockenen" Vergärung wird das zu vergärende Material nicht in eine flüssige Phase eingerührt, wie das zum Beispiel bei der Flüssigvergärung von Bioabfällen der Fall ist. Stattdessen wird das in den Fermenter eingebrachte Gärsubstrat ständig feucht gehalten, indem das Perkolat am Fermenterboden abgezogen und über der Biomasse wieder versprüht wird. So werden optimale Lebensbedingungen für die Bakterien erreicht. Bei der Rezirkulation des Perkolats kann zusätzlich die Temperatur reguliert werden, und es besteht die Möglichkeit, Zusatzstoffe für eine Prozessoptimierung zuzugeben.

Aus der WO 02/06439 ist ein Bioreaktor bzw. ein Fermenter in Form einer Fertiggarage bekannt, der nach dem Prinzip der Trockenfermentation im sogenannten Batch-Verfahren betrieben wird. Hierbei wird nach einer Animpfung mit bereits vergorenem Material wird das Gärsubstrat mit Radladern in den Fermenter gefüllt. Der garagenförmig aufgebaute Gärbehälter wird mit einem gasdichten Tor verschlossen. Die Biomasse wird unter Luftabschluss vergoren, dabei erfolgt keine weitere Durchmischung und es wird kein zusätzliches Material zugeführt. Das aus dem Gärgut sickernde Perkolat wird über eine Drainagerinne abgezogen, in einem Tank zwischengespeichert und zur Befeuchtung wieder über dem Gärsubstrat versprüht. Der Gärprozess findet im mesophilen Temperaturbereich bei 34-37°C statt, die Temperierung erfolgt mittels einer Bodenund Wandheizung.

Auch aus DE 3341691 A1 ist ein Verfahren und eine Vorrichtung zur aneroben Behandlung organischer Substanzen mit hohem Feststoffanteil bekannt, bei dem auch Biogas entsteht. Hierbei wird die Biomasse einer Rinne walkenden, peristaltischen Bewegung unterworfen und damit durch den Faulbehälter befördert. Die peristaltische Bewegung wird dadurch erreicht, dass die Ränder der Rinne parallel zueinander eine auf- und abgerichtete Bewegung ausführen. Gleichzeitig werden bei der Auf- und Abbewegung von unten Taschen zur Erzeugung der walkenden Bewegung in die Rinne eingedrückt.

Das entstehende Biogas kann in einem Blockheizkraftwerk zur Gewinnung von Strom und Wärme genutzt werden. Damit immer genug Biogas für das Blockheizkraftwerk zur Verfügung steht, werden in der Trockenfermentationsanlage mehrere Gärbehälter zeitlich versetzt betrieben. Am Ende der Verweilzeit wird der Fermenterraum vollständig entleert und dann neu befüllt. Das vergorene Substrat wird einer Nachkompostierung zugeführt, so dass ein konventionellen Komposten vergleichbarer organischer Dünger entsteht. Eine derartige Anlage läuft zur Zeit in München im Probetrieb.

Ein wesentlicher Nachteil der bekannten Großfermenter besteht darin, daß sie lediglich im Batchbetrieb arbeiten, d.h. die Biogasproduktion des Fermenters muß zum Beund Entladen unterbrochen werden und der mit Biogas gefüllte Fermenter muß mit Luft geflutet werden. Es wäre daher ein Großfermenter wünschenswert, bei dem kontinuierlich frische Biomasse zu- und verbrauchte Biomasse abgeführt wird, ohne daß die Erzeugung von Biogas unterbrochen wird. Hierzu ist es notwendig in dem Großfermenter eine Transportvorrichtung vorzusehen, mittels der die Biomasse von einem Beladebereich zu einem Entladebereich transportiert wird.

Aus der WO 93/17091 ist eine geschlossene Kompostiervorrichtung bekannt, bei der im Behälterboden mit Druckluft beaufschlagbare Blasen angeordnet sind, um die Biomasse im Behälter zu durchmischen und um sie durch den Behälter hindurch von einem Beladebereich zu einem Entnahmebereich zu transportieren. Für die Erzeugung von Biogas unter Luftabschluß ist dieses Transportverfahren aufgrund der damit verbundenen Dichtigkeitsproblem ungeeignet.

Es ist daher Aufgabe der vorliegenden Erfindung eine geeignete Transportvorrichtung für Biomasse in einem Fermenter zur Erzeugung von Biogas bereitzustellen sowie einen Großfermenter mit einer solchen Transportvorrichtung anzugeben.

Die Lösung dieser Aufgabe erfolgt durch die Merkmale des Anspruchs 1 bzw. 9.

Die erfindungsgemäße Transportvorrichtung umfaßt eine Mehrzahl von in Transportrichtung hintereinander angeordneten Transportkissen, die sukzessive mit einer Flüssigkeit gefüllt und wieder entleert werden, wodurch eine Wellenbewegung erzeugt wird, die die Biomasse transportiert.

Durch die Verwendung von Flüssigkeit anstelle von Druckluft wird sicher verhindert, dass schädliche Luft in den Faulbehälter gelangt. Darüber hinaus ergibt sich bei der Verwendung von Flüssigkeit anstelle von Druckluft oder Gas der Vorteil, dass aufgrund der Inkompressibilität von Flüssigkeiten das Volumen der in das jeweilige Transportkissen eingepumpten Flüssigkeit gleich der Volumenänderung des Transportkissens ist. Damit ergibt sich unabhängig von dem Gewicht der darüberliegenden Biomasse ein definierter "Transporthub".

Vorzugsweise - Anspruch 2 - werden die Transportkissen durch Perkolat in ihrem Volumen vergrößert und wieder verkleinert. Da ohnehin eine Perkolatzirkuliereinheit vorhanden sein muß, wird vorzugsweise aus diesem Perkolatstrom die nötige Flüssigkeit zum Betreiben der Transportkissen verwendet.

Gemäß der weiteren vorteilhaften Ausgestaltung der Erfindung nach Anspruch 3 sind die einzelnen Transportkissen unmittelbar aneinanderstoßend angeordnet. Hierdurch wird eine quasi-kontinuierliche Wellenbewegung unterstützt.

Durch das Vorsehen einer Transportkissenabdeckung nach Anspruch 4 wird verhindert, dass sich zwischen den Transportkissen Biomasse einlagert und dort verbleibt. Die Transportkissenabdeckung kann eine gliedrige Struktur haben oder aber auch lediglich aus einer Abdeckfolie bestehen.

Die Transportkissen sind gemäß der bevorzugten Ausführungsform nach Anspruch 5 auf der Bodenplatte des Faulbehälters angeordnet und befestigt. Durch das Auf und Ab der Transportkissen wird über die gesamte Breite des Transportkanals die die Biomasse transportierende Wellenbewegung erzeugt.

Alternativ oder zusätzlich können die Transportkissen auch paarweise einander zugeordnet und gegenüberliegend an den Seitenwänden angeordnet sein. Auch hierdurch wird eine peristaltische Bewegung der Biomasse durch den Transportkanal erreicht - Anspruch 6.

Je nach Trockensubstanzgehalt der Biomasse kann eine Wellenbewegung in Transportrichtung oder eine gegenläufige Wellenbewegung vorteilhaft sein. Durch ein periodisches Umschalten der Wellenbewegungsrichtung kann auch eine Durchmischung der Biomasse erreicht werden - Ansprüche 7 und 8.

Diese Transporteinrichtung läßt sich in herkömmliche Bioreaktoren oder Fermenter einbauen, wie sie beispielsweise aus der WO 02/06439 A bekannt sind, wodurch sich erfindungsgemäße Großfermenter nach Anspruch 9 ergeben. Bei den Großfermentern gemäß Anspruch 9 wird frische Biomasse in einem Beladebereich über eine Schleuse in den ständig Biogas produzierenden Großfermenter eingebracht. Im Großfermenter wird die Biomasse durch die Transporteinrichtung von dem Beladebereich zu einem Entladebereich transportiert. Während des Transports der Biomasse entsteht Biogas und die Biomasse wird "verbraucht". Im Entladebereich wird die "verbrauchte" Biomasse über eine Schleuße entnommen. Damit ist auch bei der Erzeugung von Biogas nach dem Prinzip der Feststoffmethanisierung ein kontinuierlicher Betrieb möglich.

Insbesondere bei Großfermentern zur Erzeugung von Biogas aus Biomasse treten häufig Dichtigkeitsprobleme, insbesondere an den Ecken und Kanten der Behälter sowie an den Öffnungen zum Be- und Entladen auf. Aus dem Bereich der Naßvergärung, bei der die verflüssigte Biomasse in und aus dem Faulbehälter gepumpt werden kann, sind daher Rundbehälter bekannt, die weniger Ecken und Kanten mit Dichtigkeitsproblemen aufweisen. Bei der Feststoffmethanisierung in Großfermentern werden diese Rundbehälter aufgrund der Probleme beim Be- und Entladen im BatchBetrieb nicht eingesetzt. Durch die Transportvorrichtung gemäß der vorliegenden Erfindung und den damit möglichen kontinuierlichen Betrieb lassen sich in vorteilhafterweise auch bei der "Trockenvergärung" Rundbehälter einsetzen - Anspruch 10.

Durch die Rundbauweise des Großfermenters werden Dichtungsprobleme erheblich verringert, da die Außenwand bzw. die Innenwand lediglich auf Druck bzw. Zug belastet werden. Die üblichen Dichtigkeitsprobleme von Ecken und Kanten werden jedoch vermieden. Durch die Konstruktion mit einer kreisringförmigen Innenwand und einer die Innenwand umgebenden kreisringförmigen Außenwand ergibt sich ein ringförmiger Fermenterbehälter mit einem ringförmigen Transportkanal. Dieser Kreisringzylinder wird durch eine Trennwand unterteilt. Die Biomasse wird auf einer Seite der Trennwand in einem Beladebereich kontinuierlich zugeführt und auf der anderen Seite der Trennwand am Ende des Transportkanals in einem Entladebereich kontinuierlich abgeführt. Die Zufuhr der frischen Biomasse im Beladebereich und die Entnahme der verbrauchten Biomasse im Entladebereich erfolgt über Schleusen, beispielsweise durch ein Flüssigkeitsbad nach Art eines Siphon.

Vorzugsweise wird als Transportvorrichtung eine Transportvorrichtung gemäß der vorliegenden Erfindung eingesetzt. Die notwendige Fluidsteuereinrichtung zur Ansteuerung der einzelnen Transportkissen ist mit der Perkolatzirkuliereinrichtung gekoppelt und ist vorzugsweise im Inneren innerhalb der kreisringförmigen Innenwand angeordnet -Anspruch 12.

Insbesondere bei der Verwendung von nachwachsenden Rohstoffen als Biomasse kann der hohe Flüssigkeitsgehalt der Biomasse zu einer zu starken Verflüssigung der Biomasse in dem Faulbehälter führen. Dies würde zu einer starken Beeinträchtigung der Transportwirkung der Transportvorrichtung mit Transportkissen führen. Gemäß der vorteilhaften Ausgestaltung der Erfindung nach Anspruch 11 wird nur teilvergorene Biomasse zwischen dem Be- und Entladebereich entnommen, entwässert und wieder in in den Fermenter zurückgeführt. Durch diese Maßnahme nach Anspruch 13 wird die Konzentration (Anzahl pro Volumeneinheit) von Methangas erzeugender Mikroorganismen deutlich erhöht, was sich positiv auf die weitere Biogaserzeugung auswirkt - "biologischer Turbolader-Effekt".

Die übrigen Unteransprüche beziehen sich auf vorteilhafte Ausgestaltungen der Erfindung.

Weitere Einzelheiten, Merkmale und Vorteile der Erfindung zeigt die nachfolgende Beschreibung beispielhafter Ausführungsformen anhand der Zeichnungen:

Es zeigt:
Fig. 1 einen schematische Darstellung eines langgestreckten Großfermenters dessen Boden mit Transportkissen bedeckt ist;
Fig. 2a -2c Schnittdarstellungen zur Veranschaulichung der durch die Transportkissen erzeugten Wellenbewegung;
Fig. 3a -3c Schnittdarstellungen zur Veranschaulichung der durch die Transportkissen erzeugten Wellenbewegung mittels einer alternativen Ansteuerung;
Fig. 4 eine alternative Ausgestaltung der Transportkissen;
Fig. 5 eine den Fig. 2a bis 2c und 3 entsprechende Darstellung, wobei die Transportkissen mit einer Abdeckung versehen sind, die die Wellenbewegung an die darüberliegende Biomasse weitergeben;
Fig. 6 eine Detaildarstellung der Transportkissenabdeckung;
Fig. 7 eine schematische Darstellung eines Großfermenters in Rundbauweise gemäß der vorliegenden Erfindung;
Fig. 8 eine Aufsicht auf den Boden des runden Großfermenters mit entsprechend geformten Transportkissen; und
Fig. 9 eine alternative Ausgestaltung und Anordnung der Transportkissen.

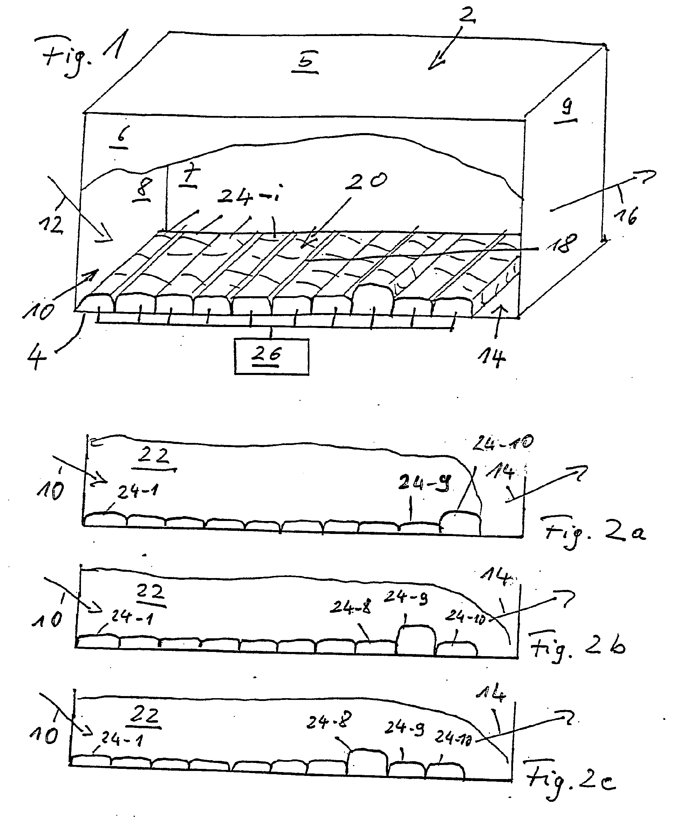

Fig. 1 zeigt schematisch einen langgestreckten, quaderförmigen Großfermenter 2 mit einer rechteckigen Bodenplatte 4, eine Deckenwand 5, einer rechten Seitenwand 6, einer linken Seitenwand 7, einer Stirnwand 8 und einer Rückwand 9. Der Großfermenter 2 umfaßt an einem Ende eine Beladebereich 10 mit einer die Stirnwand 8 durchsetzenden Beladeeinrichtung 12 - angedeutet durch einen Pfeil - und am anderen Ende einen Entladebereich 14 mit einer die Rückwand 9 durchsetzenden Entladeeinrichtung 16 - ebenfalls durch einen Pfeil bezeichnet. Zwischen Beladebereich 10 und Entladebereich 14 ist ein durch die beiden Seitenwände 6 und 7 begrenzter Transportkanal 18 ausgebildet. Der Transportkanal 18 ist mit einer Transporteinrichtung 20 versehen. Mittels der Beladeeinrichtung 12 wird im Beladebereich 10 frische Biomasse 22 kontinuierlich zugeführt. Durch die Transporteinrichtung 20 wird die Biomasse 22 zum anderen Ende des Großfermenters 2 zum Entladebereich 14 befördert. Aus dem Entladebereich 14 wird Biomasse 22 mittels der Entladeeinrichtung 16 entnommen.

Die Transporteinrichtung 20 besteht aus einer Mehrzahl von unmittelbar benachbart zueinander im Transportkanal 18 auf der Bodenplatte 4 angeordneten Transportkissen 24-i. Wie aus den Fig. 1 zu ersehen ist, erstrecken sich die einzelnen Transportkissen 24-i über die gesamte Breite des Großfermenters 2 und haben die Form von halben Ovalzylindern. Die Ausdehnung der einzelnen Transportkissen 14-i nach oben kann durch periodische Zufuhr und Entnahme von Flüssigkeit mittels einer Fluidsteuereinrichtung 26 periodische vergrößert und verkleinert werden. Durch die Zufuhr zu und Entnahme von Flüssigkeit aus unmittelbar benachbarten Transportkissen 24-i läßt sich eine Wellenbewegung erzeugen, durch die die Biomasse 22 von dem Beladebereich 10 zu dem Entladebereich 14 befördert wird.

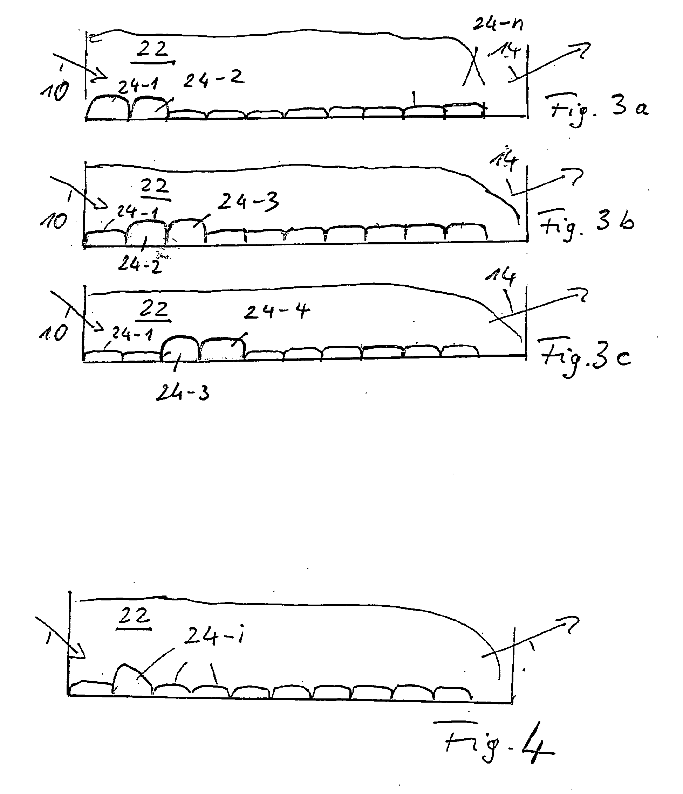

Der kontinuierliche Transport von Biomasse 22 durch die Transportkissen 24-i ist in den Figuren 2a bis 2c und 3a bis 3c für einen mit Biomasse 22 gefüllten Großfermenter 2 schematisch dargestellt. Die Figuren 2 und 3 zeigen zur Veranschaulichung der transportierenden Wellenbewegung jeweils zehn über den Transportkanal 18 verteilte Transportkissen 24-1 bis 24-10. Im Entladebereich 14 ist kein Transportkissen vorgesehen. Die Darstellung in den Figuren 3 und 4 ist schematisch und ist insbesondere nicht maßstabsgetreu.

Zunächst wird gemäß Fig. 2a in das letzte Transportkissen 24-10 vor dem Entladebereich 14 Flüssigkeit gegen das Gewicht der auf dem letzten Transportkissen 24-10 lagernden Biomasse 22 gepumpt und die auf dem letzten Transportkissen 24-10 lagernde Biomasse 22 wird angehoben und stürzt teilweise in den freien Entladebereich 14. Danach läßt man die Flüssigkeit aus dem letzten Transportkissen 24-10 durch das Gewicht der darüber lagernden Biomasse 22 wieder entweichen. Gemäß Fig. 2b wird als nächstes Flüssigkeit in das vorletzte Transportkissen 24-9 gepumpt und wieder abgelassen. Daraufhin wird das Transportkissen 24-8 aufgepumpt und wieder entleert - Fig. 2c - bis schließlich das erste Transportkissen 24-1 aufgepumpt und wieder entleert wird (nicht dargestellt). Danach beginnt der Vorgang wieder beim letzten Transportkissen 24-10. Auf diese Weise wird eine Wellenbewegung erzeugt, die die Biomasse 22 von dem Beladebereich 10 kontinuierlich zu dem Entladebereich 14 befördert. Bei dieser Ansteuerung der einzelnen Transportkissen 24-i läuft die Wellenbewegung entgegen der Transportrichtung. Diese Ansteuerung dürfte bei sehr hohem Trockensubstanzanteil der Biomasse 22 besonders geeignet sein.

Bei Biomasse 22 mit geringerem Trockensubstanzanteil und gegebenenfalls einem Flüssigkeitsspiegel über den Transportkissen in dem die Trockensubstanz der Biomasse schwimmt, dürfte eine Wellenbewegung in Transportrichtung vorteilhafter sein. Dies ist in den Fig. 3a bis 3c schematisch dargestellt. Zunächst wird gemäß Fig. 3a in das erste Transportkissen 24-1 im Beladebereich 10 Flüssigkeit gegen das Gewicht der auf dem ersten Transportkissen 24-1 lagernden Biomasse 22 gepumpt und die Flüssigkeit über dem ersten Transportkissen 24-1 wird verdrängt. Als nächstes wird das zweite Transportkissen 24-2 mit Flüssigkeit aufgepumpt - siehe Fig. 3a. Dann wird - siehe Fig. 3b - die Flüssigkeit aus dem ersten Transportkissen 24-1 abgelassen und gleichzeitig wird das dritte Transportkissen 24-3 aufgepumpt, während das zweite Transportkissen 24-2 aufgepumpt bleibt. Als nächstes - siehe Fig. 3c - wird die Flüssigkeit aus dem zweiten Transportkissen abgelassen und das vierte Transportkissen 24-4 wird aufgepumpt, während das dritte Transportkissen aufgepumpt bleibt. Auf diese Weise wird eine "Transportwelle" in Transportrichtung erzeugt, die die Biomasse 22 aus dem Beladebereich 10 zu dem Entladebereich 14 befördert.

Fig. 4 zeigt schematisch eine alternative Ausgestaltung der Transportkissen 24-i derart, dass die Oberfläche der Transportkissen 24-i in aufgepumpten Zustand in Transportrichtung geneigt ist. Durch diese Ausgestaltung wird die Förderwirkung erhöht.

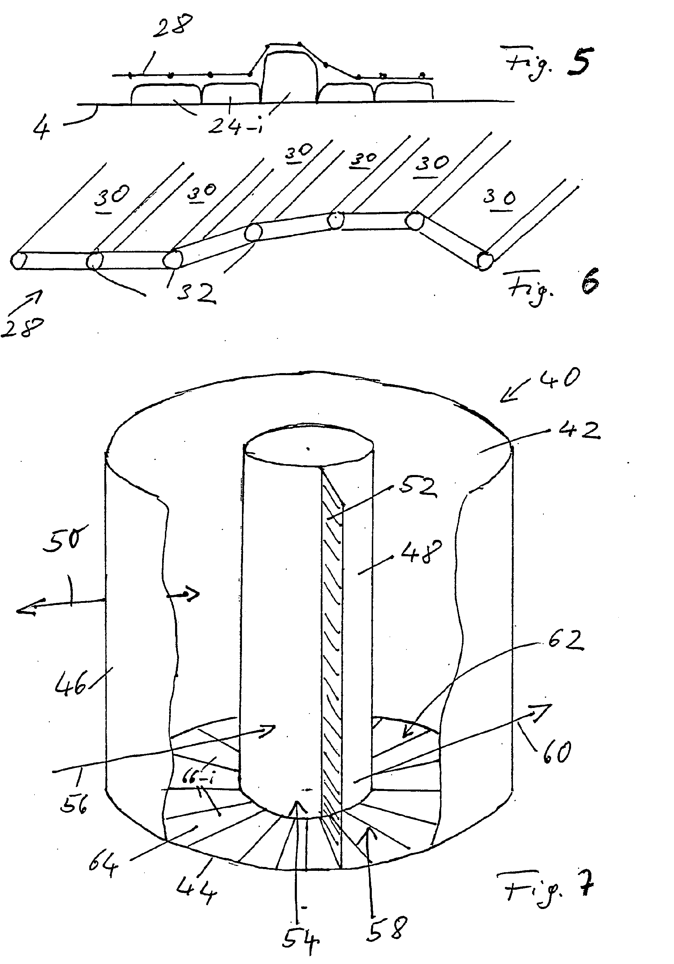

Fig. 5 zeigt eine weitere Ausgestaltung der erfindungsgemäßen Transporteinrichtung, die sich von den vorhergehend beschriebenen Ausführungsformen dadurch unterscheidet, dass die Biomasse 22 nicht unmittelbar auf den Transportkissen 24-i aufliegt, sondern auf einer Transportkissenabdeckung 28, die auf den Transportkissen 24-i aufliegt. Wie aus Fig. 5 zu ersehen ist, besteht die Abdeckung 28 besteht aus einer Mehrzahl Brettelementen 30, die über Scharnier- oder Gelenkverbindungen 32 nach Art eines Fensterrolladens gelenkig miteinander verbunden sind.

Fig. 7 zeigt einen Großfermenter 40 in Rundbauweise mit einem kreiszylindrischem Faulbehälter 42. Der Großfermenter 40 umfaßt eine ebene Bodenplatte 44. Von der Bodenplatte 44 erstreckt sich eine kreiszylinderische Außenwand 46 in die Höhe. Die kreiszylindrische Außenwand 46 umschließt eine kreiszylinderische Innenwand 48 mit kleinerem Durchmesser. Durch eine nicht dargestellte Abdeckung wird der Raum zwischen Außen- und Innenwand 46, 48 geschlossen. Die gasdicht miteinander verbundene Bodenplatte 44, die Außenwand 46, die Innenwand 48 und die Abdeckung bilden den Faulbehälter 42.

Der Faulbehälter 42 wird im Inneren durch eine Trennwand 52 unterteilt. Auf einer Seite der Trennwand 52 ist ein Beladebereich 54 mit einer die Außenwand 46 durchsetzenden Beladeeinrichtung 56 vorgesehen. Auf der anderen Seite der Trennwand 52 ist ein Entladebereich 58 mit einer die Außenwand 46 durchsetzenden Entladeeinrichtung 60 vorgesehen.

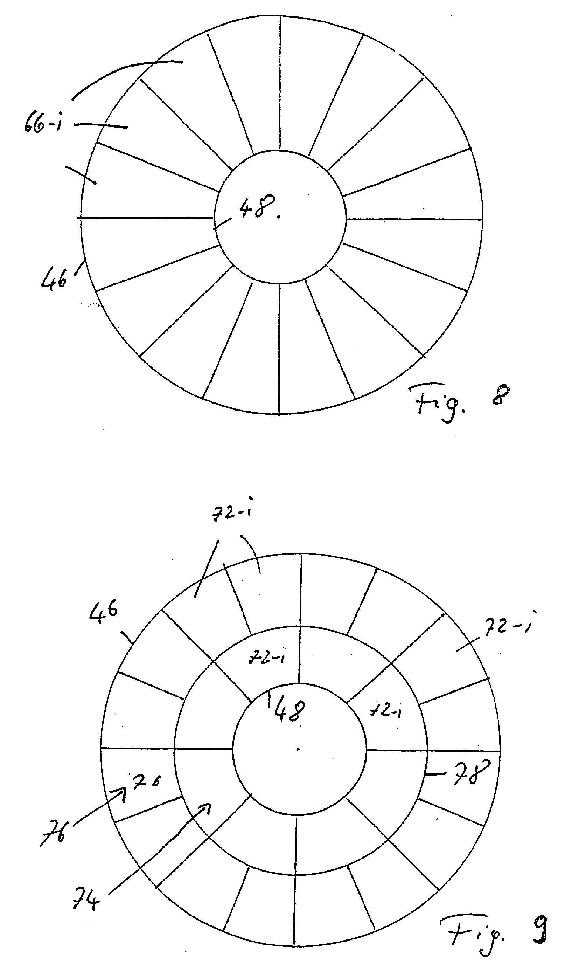

Zwischen Beladebereich 54 und Entladebereich 58 ist ein durch die Innenwand 48 und die Außenwand 46 begrenzter ringförmig verlaufender Transportkanal 62 ausgebildet. Im Transportkanal 62 ist eine Transporteinrichtung 64 der anhand der Figuren 2 bis 5 beschriebenen Art vorgesehen, die eine Mehrzahl von Transportkissen 66-i umfaßt, die auf der Bodenplatte 54 unmittelbar benachbart zueinander angeordnet sind. Wie aus Fig. 8 zu ersehen ist, besitzen die Transportkissen 66-i in etwa die Form von Kuchenstücken mit gekappter Spitze, d. h. sie sind im Bereich der Außenwand 46 breiter als im Bereich der Innenwand 48.

Der Doppelpfeil 50 bezeichnet in Fig. 7 eine Ent- und Beladeeinrichtung, die zwischen dem Beladebereich 54 und dem Entladebereich 58 die Außenwand 46 durchdringend angeordnet ist. Über die Ent- und Beladevorrichtung 50 wird die halbvergoren Biomasse 22 aus dem Faulbehälter 42 entnommen, entwässert und wieder in den Faulbehälter 42 zurückgeführt. Die Entwässerung kann beispielsweise mittels eines Separators erfolgen.

Die Transporteinrichtung 64 mit den Transportkissen 66-i ist Fig. 8 in einer Ansicht von oben dargestellt. Die transportierende Wellenbewegung wird in analoger Weise zu den Ausführungsformen gemäß den Figuren 1 bis 6 erzeugt.

Fig. 9 zeigt eine alternative Ausführungsform einer Transporteinrichtung 70 in einer Fig. 7 entsprechenden Darstellung. Die Transporteinrichtung 7 umfaßt ebenfalls eine Mehrzahl von Transportkissen 72-i, die in einem inneren Transportkanalring 74 und einem äußeren Transportkanalring 76 verteilt sind. Der innere und der äußere Transportkanalring 74, 76 werden durch eine konzentrisch zu der Innen- und Außenwand 48, 46 angeordneten Mittelwand 78 voneinander getrennt.

Dabei ist die Anzahl der Transportkissen 72-i im äußeren Transportkanalring 76 größer als im inneren Transportkanalring 74. In dem in Fig. 8 gezeigten Ausführungsbeispiel ist die Anzahl der Transportkissen 72-i im äußeren Transportkanalring 76 doppelt so groß wie inneren Transportkanalring 74. Hierdurch wird dem Umstand Rechnung getragen, dass Transportweg im äußeren Transportkanalring 76 länger ist als im inneren Transportkanalring 74. Die transportierende Wellenbewegung wird in analoger weise zu den Ausführungsformen gemäß den Figuren 1 bis 6 erzeugt.

Auch bei den Transporteinrichtungen 64 und 70 kann eine Transportkissenabdeckung gemäß den Figuren 4 und 5 vorgesehen werden. Auch die Ausgestaltung der Oberseite der Transportkissen gemäß Fig. 5 kann vorgesehen werden.

Die erfindungsgemäßen Großfermenter für den kontinuierlichem Betrieb eignen sich besonders für Biomasse aus nachwachsenden Rohstoffen, da diese aufgrund ihrer Homogenität durch die erfindungsgemäße Transporteinrichtung leicht zu befördern ist.

### Bezugszeichenliste

- 2: Großfermenter
- 4: Bodenplatte
- 5: Deckenwand
- 6: linke Seitenwand
- 7: rechte Seitenwand
- 8: Stirnwand
- 9: Rückwand
- 10: Beladebereich
- 12: Beladeeinrichtung
- 14: Entladebereich
- 16: Entladeeinrichtung
- 18: Transportkanal
- 20: Transporteinrichtung
- 22: Biomasse
- 24-i: Transportkissen
- 26: Fluidsteuereinrichtung
- 28: Transportkissenabdeckung
- 30: Brettelemente
- 32: Scharnier- oder Gelenkverbindung

- 40: Großfermenter in Rundbauweise
- 42: Faulbehälter
- 44: Bodenplatte
- 46: Außenwand
- 48: Innenwand
- 50: Ent- und Beladeeinrichtung
- 52: Trennwand
- 54: Beladebereich
- 56: Beladeeinrichtung
- 58: Entladebereich
- 60: Entladeeinrichtung
- 62: Transportkanal
- 64: Transporteinrichtung
- 66-i: Transportkissen

- 70: Transporteinrichtung
- 72-i: Transportkissen
- 74: innerer Transportkanalring
- 76: äußerer Transportkanalring
- 78: Mittelwand

## Patentansprüche

1. Transporteinrichtung für Biomasse (22), insbesondere aus nachwachsenden Rohstoffen, in einem Fermenter (2; 40) zur Erzeugung von Biogas, mit einem Transportkanal (18; 62) mit einander gegenüberliegenden Seitenwänden (6, 7; 46, 48), die über eine Bodenfläche (4) miteinander verbunden sind, einer Mehrzahl von über den Transportkanal (18; 62) verteilten, mit Fluid befüllbaren Transportkissen (14-i; 66-i; 72-i), die unter der zu transportierenden Biomasse (22) angeordnet sind, und
einer Fluidsteuereinrichtung (26) zum sukzessiven Befüllen und Entleeren aufeinanderfolgender Transportkissen (24-i; 66-i; 72-i), wodurch eine Wellenbewegung erzeugbar ist, durch die die Biomasse (22) durch den Transportkanal (18; 62) bewegt wird, **dadurch gekennzeichnet,**
**dass** das Fluid zum Befüllen der Transportkissen eine Flüssigkeit ist.

2. Transporteinrichtung nach Anspruch 1, **dadurch gekennzeichnet, dass** die Flüssigkeit zum Befüllen der Transportkissen (24-i; 66-i; 72-i) Perkolat ist.

3. Transporteinrichtung nach einem der vorhergehenden Ansprüche, **dadurch gekennzeichnet, dass** die Transportkissen (24-i; 66-i; 72-i) aneinanderstoßend angeordnet sind.

4. Transporteinrichtung nach einem der vorhergehenden Ansprüche, **dadurch gekennzeichnet, dass** über den Transportkissen (24-i; 66-i; 72-i) eine Transportkissenabdeckung (28) vorgesehen ist, welche die durch die Transportkissen (24-i; 66-i; 72-i) erzeugbare wellenbewegung auf die Biomasse (22) überträgt.

5. Transporteinrichtung nach einem der vorhergehenden Ansprüche, **dadurch gekennzeichnet, dass** die Transportkissen (24-i; 66-i; 72-i) auf der Bodenfläche (4) befestigt sind.

6. Transporteinrichtung nach Anspruch 3 oder 4, **dadurch gekennzeichnet, dass** die Transportkissen paarweise einander zugeordnet sind und gegenüberliegend an den Seitenwänden (6, 7; 46, 48) befestigt sind.

7. Transporteinrichtung nach einem der vorhergehenden Ansprüche, **dadurch gekennzeichnet, dass** durch die Transportkissen (20; 64) eine Wellenbewegung entgegen der Transportrichtung erzeugbar ist.

8. Transporteinrichtung nach einem der vorhergehenden Ansprüche, **dadurch gekennzeichnet, dass** durch die Transportkissen (20; 64) eine Wellenbewegung in Transportrichtung erzeugbar ist.

9. Großfermenter zur Erzeugung von Biogas aus Biomasse nach dem Prinzip der Feststoffmethanisierung, mit einem gasdichten Faulbehälter (42) mit einer Bodenplatte (4; 44),
einem Beladebereich (10; 54) mit einer den gasdichten Faulbehälter (42) durchsetzenden Beladeeinrichtung (12; 56) zum kontinuierlichen Zuführen von frischer Biomasse (22) in den Faulbehälter (42),
einem Entladebereich (14; 58) mit einer den gasdichten Faulbehälter (42) durchsetzenden Entladeeinrichtung (16; 58) zum Entnehmen von verbrauchter Biomasse (22) aus dem Faulbehälter (42),
einer Transporteinrichtung (20; 64; 70) nach einem der vorhergehenden Ansprüche zum Transportieren der Biomasse (22) von dem Beladebereich (10; 54) zu dem Entladebereich (14, 58), und
einem Biogasentnahmeanschluß.

10. Großfermenter nach Anspruch 9, **dadurch gekennzeichnet, dass** der Faulbehälter (42) umfaßt:
eine gasdichte, sich von der Bodenplatte (44) in die Höhe erstreckende zylinderische, insbesondere kreiszylinderische Außenwand (46),
eine gasdichte, sich von der Bodenplatte (44) in die Höhe erstreckende zylinderische, insbesondere kreiszylinderische Innenwand (48),
eine gasdichten Abdeckung, die mit Außen- und Innenwand (46, 48) verbunden ist,
wodurch zwischen Bodenplatte (44), Außenwand (46), Innenwand (48) und Abdeckung Faulbehälter (42) mit einer ringzylindrischen Form zur Aufnahme der Biomasse (22) festgelegt ist,
eine sich von der Bodenplatte (44) in die Höhe erstreckenden Trennwand (52),
wobei die Beladeeinrichtung (56) auf einer Seite der Trennwand (52) die Außenwand (46) durchsetzt, und
wobei die Entladeeinrichtung (58) auf der anderen Seite der Trennwand (52) die Außenwand (46) durchsetzt.

11. Großfermenter nach Anspruch 9, **dadurch gekennzeichnet, dass** sich zwischen Innenwand (48) und Außenwand (46) ein Mittelwand (78) sich von der Bodenplatte () in die Höhe erstreckt, wodurch zwischen Innenwand (48) und Mittelwand (78) ein innerer Transportkanalring (74) und zwischen Außenwand (46) und Mittelwand ein äußerer Transportkanalring (76) gebildet wird.

12. Großfermenter nach einem der Ansprüche 9 bis 11, **gekennzeichnet durch** eine Perkolatzirkuliereinrichtung, die einen Perkolatvorratsbehälter aufweist, der innerhalb der zylinderischen Innenwand (48) angeordnet ist.

13. Großfermenter nach einem der Ansprüche 9 bis 12, **dadurch gekennzeichnet, dass** wenigstens eine Ent- und Beladeeinrichtung (50) vorgesehen ist, die zwischen dem Beladebereich (10; 54) und dem Entladebereich (14; 58) angeordnet ist, um Biomasse aus dem Faulbhälter (2; 40, 42) zu entnehmen, zu entwässern und wieder dem Faulbehälter zuzuführen.

## Claims

1. A transporting arrangement for biomass (22), in particular from renewable resources, in a fermenter (2; 40) for generating biogas, comprising a transporting channel (18; 62) having mutually opposed side walls (6, 7; 46, 48) which are connected to each other via a bottom surface (4),
a plurality of transporting cushions (24-i; 66-i; 72-i) which are distributed over the transporting channel (18; 62), adapted to be filled with fluid, and arranged underneath the biomass (22) to be transported, and
a fluid control arrangement (26) for successively filling and emptying consecutive transporting cushions (24-i; 66-i; 72-i), thus enabling the generation of an undulating movement whereby the biomass (22) is moved through the transporting channel (18; 62),
**characterized in that**
the fluid for filling the transporting cushions is a liquid.

2. The transporting arrangement as claimed in claim 1, **characterized in that** the liquid for filling the transporting cushions (24-i; 66-i; 72-i) is percolate.

3. The transporting arrangement as claimed in any one of the preceding claims, **characterized in that** the transporting cushions (24-i; 66-i; 72-i) are arranged in abutment against one another.

4. The transporting arrangement as claimed one of the preceding claims, **characterized in that** a transporting cushion cover (28) is provided over the transporting cushions (24-i; 66-i; 72-i), whereby the undulating movement which can be generated by the transporting cushions (24-i; 66-i; 72-i) is transmitted to the biomass (22).

5. The transporting arrangement as claimed in any one of the preceding claims, **characterized in that** the transporting cushions (24-i; 66-i; 72-i) are fastened on the bottom surface (4).

6. The transporting arrangement as claimed in claim 3 or 4, **characterized in that** the transporting cushions are assigned to one another in pairs and are fastened opposite one another on the side walls (6, 7; 46, 48).

7. The transporting arrangement as claimed in any one of the preceding claims, **characterized in that** an undulating movement opposite to the transporting direction can be generated by the transporting cushions (20; 64).

8. The transporting arrangement as claimed in any one of the preceding claims, **characterized in that** an undulating movement in the transporting direction can be generated by the transporting cushions (20; 64).

9. A large-scale fermenter for generating biogas from biomass in accordance with the principle of methanation of solid matter, comprising
a gas-tight digestion tank (42) having a base plate (4; 44),
a loading region (10; 54) including a loading arrangement (12; 56) which extends through the gas-tight digestion tank (42) and is intended for continuously feeding fresh biomass (22) into the digestion tank (42),
an unloading region (14; 58) including an unloading arrangement (16; 58) which extends through the gas-tight digestion tank (42) and is intended for removing spent biomass (22) from the digestion tank (42),
a transporting arrangement (20; 64; 70) as claimed in any one of the preceding claims for transporting the biomass (22) from the loading region (10; 54) to the unloading region (14, 58), and
a biogas removal port.

10. The large-scale fermenter as claimed in claim 9, **characterized in that** the digestion tank (42) comprises:
a gas-tight, cylindrical, in particular circular-cylindrical, outer wall (46) which extends upwards from the base plate (44),
a gas-tight, cylindrical, in particular circular-cylindrical, inner wall (48) which extends upward from the base plate (44),
a gas-tight covering which is connected to the outer and inner walls (46, 48),
as a result of which a cylindrical digestion tank (42) for accommodating the biomass (22) is defined between base plate (44), outer wall (46), inner wall (48) and covering,
a partition wall (52) which extends upward from the base plate (44),
the loading arrangement (56) extending through the outer wall (46) on one side of the partition wall (52), and
the unloading arrangement (58) extending through the outer wall (46) on the other side of the partition wall (52).

11. The large-scale fermenter as claimed in claim 9, **characterized in that** a central wall (78) extends upward from the base plate (44) between the inner wall (48) and the outer wall (46), as a result of which an inner transporting channel ring (74) is formed between the inner wall (48) and the central wall (78), and an outer transporting channel ring (76) is formed between the outer wall (46) and the central wall.

12. The large-scale fermenter as claimed in any one of claims 9 to 11, **characterized by** a percolate circulating arrangement having a percolate storage tank which is arranged within the cylindrical wall (48).

13. The large-scale fermenter as claimed in any one of claims 9 to 12, **characterized in that** at least one removal and loading arrangement (50) is provided which is arranged between the loading region (10; 54) and the unloading region (14; 58) in order for biomass to be removed from the digestion tank (2; 40, 42), dehydrated, and fed back to the digestion tank.

## Revendications

1. Dispositif de transport de biomasse (22), en particulier constituée de matières premières en cours de transformation, dans un digesteur (2 ; 40) pour la production de biogaz, comportant un canal de transport (18 ; 62) qui a des parois latérales se faisant face (6, 7 ; 46, 48), reliées entre elles par une surface de fond (4), comportant une pluralité de coussins de transport (24-i ; 66-i ; 72-i) remplissables de fluide, répartis sur le canal de transport (18 ; 62) et disposés sous la biomasse à transporter (22), et comportant une unité de commande des fluides (26) destinée à remplir et à vider successivement les coussins de transport consécutifs (24-i ; 66-i ; 72-i), moyennant quoi un mouvement ondulatoire peut être généré au moyen duquel la biomasse (22) est déplacée dans le canal de transport (18 ; 62), **caractérisé en ce que** le fluide destiné à remplir les coussins de transport est un liquide.

2. Dispositif de transport selon la revendication 1, **caractérisé en ce que** le liquide destiné à remplir les coussins de transport (24-i ; 66-i ; 72-i) est un percolat.

3. Dispositif de transport selon l'une des revendications précédentes, **caractérisé en ce que** les coussins de transport (24-i ; 66-i ; 72-i) sont disposés de façon à être en contact les uns avec les autres.

4. Dispositif de transport selon l'une des revendications précédentes, **caractérisé en ce qu'**est prévue sur les coussins de transport (24-i ; 66-i ; 72-i) une housse de coussins de transport (28), qui transmet à la biomasse (22) le mouvement ondulatoire pouvant être généré par les coussins de transport (24-i ; 66-i ; 72-i).

5. Dispositif de transport selon l'une des revendications précédentes, **caractérisé en ce que** les coussins de transport (24-i ; 66-i ; 72-i) sont fixés sur la surface de fond (4).

6. Dispositif de transport selon la revendication 3 ou 4, **caractérisé en ce que** les coussins de transport sont mutuellement associés par paires et sont fixés en vis-à-vis aux parois latérales (6, 7 ; 46, 48).

7. Dispositif de transport selon l'une des revendications précédentes, **caractérisé en ce qu'**un mouvement ondulatoire peut être généré par les coussins de transport (20 ; 64) dans le sens opposé à la direction de transport.

8. Dispositif de transport selon l'une des revendications précédentes, **caractérisé en ce qu'**un mouvement ondulatoire peut être généré par les coussins de transport (20 ; 64) dans le sens du transport.

9. Digesteur à grande capacité pour la production de biogaz à partir de biomasse selon le principe de la méthanisation de matières solides, comportant un fermenteur étanche aux gaz (42) avec une plaque de fond (4 ; 44),
une zone de chargement (10 ; 54) pourvue d'un dispositif de chargement (12; 56) traversant le fermenteur étanche aux gaz (42) et destiné à alimenter en continu le fermenteur (42) en biomasse (22) fraîche, une zone de déchargement (14 ; 58) pourvue d'un dispositif de déchargement (16 ; 58) traversant le fermenteur étanche aux gaz (42) et destiné à évacuer du fermenteur (42) la biomasse (22) consommée,
un dispositif de transport (20 ; 64 ; 70) selon l'une des revendications précédentes pour transporter la biomasse (22) de la zone de chargement (10 ; 54) à la zone de déchargement (14 ; 58), et
un raccord de prélèvement du biogaz.

10. Digesteur à grande capacité selon la revendication 9, **caractérisé en ce que** le fermenteur (42) comprend :
une paroi extérieure (46) étanche aux gaz, cylindrique, en particulier circulaire, s'étendant vers le haut en partant de la plaque de fond (44),
une paroi intérieure (48) étanche aux gaz, cylindrique, en particulier circulaire, s'étendant vers le haut en partant de la plaque de fond (44),
un couvercle étanche aux gaz, relié aux parois extérieure et intérieure (46, 48),
moyennant quoi le fermenteur (42), de forme cylindrique annulaire et destiné à recevoir la biomasse (22), est disposé entre la plaque de fond (44), la paroi extérieure (46), la paroi intérieure (48) et le couvercle,
une paroi de séparation (52) s'étendant vers le haut en partant de la plaque de fond (44),
dans lequel le dispositif de chargement (56) traverse la paroi extérieure (46) d'un côté de la paroi de séparation (52) et dans lequel le dispositif de déchargement (58) traverse la paroi extérieure (46) de l'autre côté de la paroi de séparation (52).

11. Digesteur à grande capacité selon la revendication 9, **caractérisé en ce qu'**une paroi intermédiaire (78) s'étend vers le haut depuis la plaque de fond (44) entre la paroi intérieure (48) et la paroi extérieure (46), moyennant quoi un canal de transport annulaire intérieur (74) est formé entre la paroi intérieure (48) et la paroi intermédiaire (78) et un canal de transport annulaire extérieur (76) est formé entre la paroi extérieure (46) et la paroi intermédiaire (78).

12. Digesteur à grande capacité selon l'une des revendications 9 à 11, **caractérisé par** un dispositif de circulation du percolat comprenant un réservoir de percolat disposé à l'intérieur de la paroi intérieure cylindrique (48).

13. Digesteur à grande capacité selon l'une des revendications 9 à 12, **caractérisé en ce qu'**est prévu au moins un dispositif de déchargement et de chargement (50), disposé entre la zone de chargement (10 ; 54) et la zone de déchargement (14 ; 58) pour prélever de la biomasse à partir du fermenteur (2 ; 40, 42), en éliminer l'eau et en alimenter à nouveau le fermenteur.
